# EUROPEAN PATENT SPECIFICATION

(11) **EP 2 222 843 B1**
(45) Date of publication and mention of the grant of the patent: **23.03.2016**
(21) Application number: 08849580.9
(22) Date of filing: 14.11.2008
(51) Int. Cl.: C12N 9/94, C12Q 1/24, C12Q 1/04

(54) **NOVEL PROCESS FOR SEPARATING AND DETERMINING THE VIRAL LOAD IN A PANCREATIN SAMPLE**
VERFAHREN ZUR ABTRENNUNG UND BESTIMMUNG DER VIRALEN BELASTUNG IN EINER PANKREATINPROBE
PROCÉDÉ POUR SÉPARER ET DÉTERMINER LA CHARGE VIRALE DANS UN ÉCHANTILLON DE PANCRÉATINE

(30) Priority: 15.11.2007 EP 07120740; 15.11.2007 US 988323 P
(43) Date of publication of application: 01.09.2010
(73) Proprietor: Abbott Laboratories GmbH, 30173 Hannover (DE)
(72) Inventor: BECHER, Dietmar, 17498 Diedrichshagen (DE); DÖHNER, Leopold, 17489 Greifswald (DE); RÜFFER, Frauke, 30173 Hannover (DE); FRINK, Martin, 30173 Hannover (DE)
(74) Representative: Hart-Davis, Jason
(86) International application number: PCT/EP2008/065586
(87) International publication number: WO 2009/063065

(56) References cited:
- EP-A- 1 593 688
- WO-A-98/38292
- WO-A-2007/014896
- WO-A-2007/135125

## Description

The present invention relates to a process for substantially quantitatively separating the viral load from a pancreatin specimen and to a process for quantitatively determining the viral load of this pancreatin specimen with high sensitivity.

Pancreatin is a well-known mixture of various physiologically active constituents obtained from mammalian pancreatic glands. The main constituents of pancreatin are digestive enzymes, in particular pancreatic lipase, but also amylases and proteases. Thanks to its valuable therapeutic properties and high level of safety in use, pancreatin has long been used, highly successfully as a pharmaceutical preparation in enzyme replacement therapy. Pancreatic lipase is of greatest significance here, but the amylases and proteases also make a considerable contribution to the therapeutic benefits of pancreatin. Pancreatin for therapeutic purposes is usually obtained from cattle ("bovine pancreatin") or pigs ("porcine pancreatin"), with porcine pancreatin being of the greatest significance in quantity terms. Methods for the production of pancreatin for therapeutic purposes are known per se, for example from the publication EP 0 115 023 A.

Due to the nature of animal-derived pancreatin, the starting materials may typically be accompanied by unwanted biological components, such as bacterial or viral contaminants. However, during more than 100 years of commercialization of pharmaceutical products containing pancreatin, no case has ever been reported where patients have been affected by viral-contaminated pancreatin. Nevertheless, companies producing pharmaceutical products derived from biological tissues and/or body fluids experience increasing pressure from the regulatory bodies to increase the level of safety of their products by reducing all contaminants to the lowest level possible, independent of whether any concerned contaminant is considered a human pathogen or not. For the application of pancreatin in pharmacological products, it is therefore desirable to have reliable analytical methods for detecting and quantifying such biological contaminants.

Until recently, no reliable method has been developed for quantitatively detecting or separating viral contaminants in a pancreatin sample. This is probably due to the fact that the enzymatically active constituents of pancreatin are incompatible with the cell lines typically used for multiplying viruses using techniques known to a person of ordinary skill in the art, thus making it more difficult or even impossible to determine the virus titer in a pancreatin sample.

Only recently, a method has been developed to allow separation and detection of the viral load in a pancreatin sample, see unpublished patent application PCT/EP2007/054880. However, since the viral load of the preparations derived from animals, like pancreatin, is very low, there is an ongoing need for providing processes having high sensitivity for separating and determining the viral load in pharmaceutical preparations, in particular, in pancreatin preparations for pharmaceutical use.

It was accordingly the object of the invention to provide a highly sensitive process for substantially quantitatively separating the viral load from a pancreatin specimen and a process for quantitatively determining the viral load of this pancreatin specimen. In particular, it was an object of the invention to provide a process for substantially quantitatively separating an infectious viral load from a pancreatin specimen and a process for quantitatively determining the infectious viral load of this pancreatin specimen.

It has now surprisingly been found that the viral load, in particular the infectious viral load, of a pancreatin specimen may be substantially quantitatively determined with high sensitivity if the viral load is first separated from the pancreatin specimen in a multistage centrifugation process and the separated viral load is then quantitatively determined using per se known virological methods.

Publication JP 12856990 has already disclosed a method for concentrating or isolating hepatitis viruses by a nonspecific combination of low-speed centrifugation and ultracentrifugation.

In a first embodiment, the invention relates to a process for separating the viral load from a pancreatin specimen, comprising the process steps:
a) producing a liquid pancreatin test sample suitable for centrifugation from the pancreatin specimen without in so doing changing the viral load thereof,
b) subjecting at least one defined part of the pancreatin test sample from process step a) to at least one low-speed centrifugation under conditions, under which viruses with sedimentation constants of ≥ 120 S do not yet form a pellet,
c) discarding any solid deposit optionally arising in process step b) during low-speed centrifugation and retaining a pancreatin test sample supernatant,
d) subjecting at least one defined part of the pancreatin test sample supernatant obtained in process step c) to a first ultracentrifugation in a discontinuous gradient medium, wherein the duration of the first ultracentrifugation and the relative centrifugal force for the first ultracentrifugation are selected such that the viral load is quantitatively transported out of the pancreatin test sample supernatant into a first target fraction situated above or in the boundary layer between the overlying, lowest concentration gradient component and the underlying, next higher concentration gradient component, and quantitatively separating the first target fraction optionally including any pellet present after centrifugation containing the viral load from the pancreatin test sample supernatant;
e) subjecting the first target fraction obtained in step d) to a second ultracentrifugation whereby said second ultracentrifugation is carried out with a relative centrifugal force higher than the relative centrifugal force of the first ultracentrifugation on a gradient medium of the same type as used in the first ultracentrifugation whereby said gradient medium is a higher concentration gradient medium than the concentration of the lowest concentration gradient component in the first target fraction, and
f) obtaining a second target fraction containing the viral load by discarding the upper 75% vol./vol. liquid or more of the upper layer corresponding to the first target fraction and obtaining the lower fraction corresponding to the lower 25% vol./vol. liquid or less of the upper layer and the complete layer of the higher concentration gradient medium excluding any pellet optionally present after centrifugation.

In a second embodiment, the invention relates to a process for quantitatively determining the viral load of the pancreatin specimen, in particular the infectious viral load of the pancreatin specimen. For example, in the second embodiment, in addition to the process according to the first embodiment, in a further process step g) to be carried out after process step f), a quantitative determination of the viral load of the pancreatin specimen is carried out by determining the virus infection titer in the target fraction containing the viral load, allowing determining the infectious viral load in the test sample.

Unless otherwise specified herein, any technical and scientific terms stated below will in each case have the same meaning as they are conventionally understood to have by a person skilled in the particular field of the art. Temperature ranges cited hereinafter in the format of e.g. "0-15°C" denominate a temperature range of from 0°C to 15°C with the range limits being included in each case. Time ranges cited hereinafter in the format of e.g. "30-120 minutes" denominate a time range of from 30 minutes to 120 minutes with the range limits being included in each case. Time ranges cited hereinafter in the format of e.g. "2-8 hours" denominate a time range of from 2 hours to 8 hours with the range limits being included in each case. Ranges of the relative centrifugal force cited hereinafter in the format of e.g. "1,500-5,000 × g" denominate a relative centrifugal force in the range of from 1,500 × g to 5,000 × g with the range limits being included in each case. Volume ranges cited hereinafter in the format of e.g. "10-15 ml" denominate a volume range of from 10 milliliters to 15 milliliters with the range limits being included in each case. Measure of length ranges cited hereinafter in the format of e.g. "80-100 mm" denominate a measure of length range of from 80 millimeters to 100 millimeters with the range limits being included in each case.

The process according to the invention is suitable for all types of pancreatin of animal origin and may in particular be carried out on conventional commercial porcine pancreatins and on bovine pancreatins. The process is preferably carried out on porcine pancreatin specimens.

The process described herein is generally suitable for separating the viral load from a pancreatin specimen and for subsequently quantitatively determining the viral load of a pancreatin specimen. In particular, the process is suitable for separating and for quantitatively determining the viral load of pancreatin specimens, in which the viral load comprises bovine rotavirus A, encephalomyocarditis virus (= EMCV), porcine circovirus (= PCV), porcine parvovirus (= PPV), porcine rotavirus A, porcine teschovirus and/or swine vesicular disease virus (= SVDV). Thanks to its very similar properties, human coxsackievirus B 5/1 may be used as a model for verifying the suitability of the process according to the invention for separating and/or quantitatively determining SVDV. Thanks to its very similar properties, bovine rotavirus A (for example strain B 223) may be used as a model for verifying the suitability of the process according to the invention for separating and/or quantitatively determining porcine rotavirus A. The process described herein is also suitable to determine the viral load with hepatitis E virus (= HEV) in a pancreatin specimen by separating the viral load as described and subsequently using further analysis using known technologies, e.g. polymerase chain reaction (= "PCR") or the like.

In process step a) of the process described herein, a liquid pancreatin test sample suitable for centrifugation is produced from the pancreatin specimen without in so doing changing or modifying the viral load, in particular the infectious viral load, thereof. This may, for example, proceed by producing a pancreatin test sample suspension from the pancreatin specimen. For example, a pancreatin test sample suspension is produced by combining the pancreatin specimen with a cell culture medium which is suitable for the cell line used to culture the virus species to be investigated and with one or more antibiotic(s) suitable for this purpose. In another embodiment, the pancreatin test sample suspension is produced by combining the pancreatin specimen with a saline solution, in particular, a phosphate-buffered saline solution (= "PBS", pH 7.2). Generally, all antibiotics are suitable for producing the antibiotic solution optionally used in process step a). As a rule, broad-spectrum antibiotics or mixtures of such broad-spectrum antibiotics are used. Suitable antibiotics may e.g. be selected from the group comprising beta-lactam antibiotics like penicillins, cephalosporins (including oxacephems and carbacephems), carbapenems and monobactams; streptomycin (including streptomycin sulfate); neomycins (including neomycin A, neomycin B and paromomycin); kanamycins (including kanamycin, gentamicin, amikacin and tobramycin); spectinomycin; tetracyclines (including tetracycline, oxytetracycline, doxycycline and minocycline); macrolide antibiotics (including erythromycin, clarithromycin, roxithromycin, azithromycin, josamycin and spiramycin); gyrase inhibitors (including nalidixic acid, cinoxacin, pipemidic acid, norfloxacin, pefloxacin, ciprofloxacin, ofloxacin and fleroxacin; folic acid antagonists (including sulfonamide antibiotics, diamino benzylpyrimidines and their combinations); chloramphenicol; lincosamides; glycopeptide antibiotics (including vancomycin and teicoplanin); fosfomycin; polypeptide antibiotics (including polymixin B, colistin, bacitracin and tyrothicin) and mupirocin. Preferred antibiotics are streptomycin sulfate and penicillin and mixtures of streptomycin sulfate and penicillin, for example as an antibiotic "cocktail". The one or more antibiotic(s) may for example be used in a solution of a solvent which is suitable for the one or more antibiotic(s) in each case, i.e. as an antibiotic solution.

The pancreatin test sample suspension is conventionally produced with cooling to a temperature of 0-15°C, for example to a temperature of 4-10°C. The constituents for producing the pancreatin test sample suspension are conventionally stirred with ice cooling and for a duration of at least 30 minutes, for example 30-120 minutes, preferably 45-90 minutes, in particular 50 minutes or 60 minutes. The purpose of cooling the pancreatin test sample suspension and of cooling in all further process steps is in each case to avoid or at least substantially reduce any unwanted deactivation of the viral load by the enzymatically active constituents of the pancreatin specimen.

In case of using cell culture medium for the preparation of the pancreatin test sample suspension, the cell culture media are in each case used in producing the pancreatin test sample suspension in process step a), is determined by which virus species is to be separated and/or quantitatively determined by the process according to the invention. Permissive cell cultures in which the investigated virus species if possible initiates a cytopathic effect (= CPE) are used for culturing and detecting a specific virus species. CPE is a modification of virus-infected cells which is recognizable by light microscopy. If a virus species multiplies in the culture cell without CPE, such multiplication may in general nevertheless be identified by per se known indirect detection methods.

If, for example, bovine rotavirus A is to be separated and/or quantitatively determined, fetal monkey kidney cells (= MA-104 cells) may, for example, be used for the culturing thereof. In this case, per se known Dulbecco's Modified Eagle Medium (= Dulbecco medium) is, for example, suitable as the cell culture medium. If EMCV is to be separated and/or quantitatively determined, porcine kidney cells (= PK-15 cells) or embryonic porcine kidney cells (= SPEV cells) may be used for the culturing thereof. In the case of PK-15 cells, per se known Minimal Essential Medium (= MEM) is, for example suitable as the cell culture medium. In the case of SPEV cells, Dulbecco medium is, for example, suitable as the cell culture medium. If, for example, PCV is to be separated and/or quantitatively determined, PK-15 cells may, for example, be used for the culturing thereof. If, for example, PPV is to be separated and/or quantitatively determined, porcine kidney cells (SK-6 cells) may, for example, be used for the culturing thereof. In this case, Dulbecco medium is, for example, suitable as the cell culture medium. If, for example, porcine rotavirus A is to be separated and/or quantitatively determined, MA-104 cells may, for example, be used for the culturing thereof. If, for example, porcine teschovirus is to be separated and/or quantitatively determined, PK-15 cells may, for example, be used for the culturing thereof. If, for example, SVDV is to be separated and/or quantitatively determined, SPEV cells may, for example, be used for the culturing thereof. The person skilled in the art is aware of suitable cell lines for culturing the particular virus species and of corresponding suitable cell culture media. Virus species usable according to the present invention and corresponding cell lines may be obtained from appropriate sources, for example from the "American Type Culture Collection", Manassas, USA (= ATCC), the "Deutsche Sammlung von Mikroorganismen und Zellkulturen GmbH", Braunschweig, Germany (= DSMZ), the "Friedrich-Löffler-Institut", Federal Research Institute for Animal Health, Insel Riems, Germany (= FLI) or the "Veterinary Service Division" of the "Department of Agriculture and Rural Development", Belfast, United Kingdom (= DARD).

In process step b), the pancreatin test sample obtained in process step a) may either be used in its entirety, or a defined part of the pancreatin test sample, in particular a defined volume of this pancreatin test sample, may be used. The pancreatin test sample obtained in process step a) is preferably used in its entirety.

In process step b), in the case of low-speed centrifugations, conditions may be established under which viruses with sedimentation constants of ≥ 120 S, in particular of ≥ 120 S to 5,000 S, do not yet form a pellet. Usually viruses with sedimentation constants of ≥ 120 S, in particular viruses with sedimentation constants of ≥ 120 S to 5,000 S, do not yet form pellets when the low-speed centrifugations are carried out with a relative centrifugal force of in each case less than 15,000 × g, preferably of in each case 8,000-15,000 × g, particularly preferably of in each case 10,000-13,500 × g, for example of in each case 10,800 × g. The duration of a low-speed centrifugation step conventionally amounts to at least 5 minutes, usually 5-60 minutes, in particular 10-45 minutes, preferably 10-30 minutes, for example 15 minutes. In a preferred embodiment of process step b), low-speed centrifugation steps are carried out with cooling to a temperature of 0-15°C, for example to a temperature of 4-10°C, preferably in a refrigerated centrifuge.

The purpose of low-speed centrifugation steps in process step b) is primarily to remove from the pancreatin suspension those pancreatin constituents such as insoluble particles etc. which are disruptive in the separation and/or quantitative determination of the viral load of a pancreatin specimen in order to obtain a pancreatin test sample supernatant which is suitable for further processing. The solid deposits optionally obtained in low-speed centrifugation steps are thus generally discarded in process step c), while the supernatant is used for the next process step d). Low-speed centrifugation steps with subsequent discarding of optionally obtained solid deposits are conventionally repeated until solid deposits are no longer observed to form. Usually, no further repetitions will be necessary after 1-3 repetitions, in particular after just one repetition, of low-speed centrifugation steps with subsequent discarding of optionally obtained solid deposits. In an embodiment of the invention, a solid deposit as obtained in process step b) can be a sediment or pellet.

In one embodiment of the invention, the deposit obtained in low-speed centrifugation steps is washed once or more, for example 1-3 times, with a suitable washing fluid before being discarded. A suitable washing fluid is, for example, the pancreatin test sample supernatant obtained after low-speed centrifugation itself, which may then be used in process step d). If a washing fluid other than the pancreatin test sample supernatant itself is used, said washing fluid is combined, once washing of the deposit is complete, with the pancreatin test sample supernatant and then used in process step d). It is particularly advantageous to wash the deposit in the above-stated manner before use in process step d), if the viral load of the pancreatin specimen comprises EMCV.

In a specific embodiment, the invention also comprises a pancreatin test sample supernatant, which may be produced according to process steps a)-c) as indicated above.

A discontinuous two-phase sucrose gradient is conventionally used as the discontinuous gradient medium in process step d). The discontinuous gradient medium preferably comprises a gradient prepared from a 50% (wt./vol.) buffered sucrose solution and a 20% (wt./vol.) buffered sucrose solution. Neutral buffers (i.e. which buffer around a pH value of 7) may, for example, be used as the buffer for the sucrose solutions. A PBS buffer is preferably used. Sterile gradient media are usually used. A two-phase discontinuous sucrose gradient of the above-stated kind provides particularly suitable sedimentation and thus separation conditions for the viruses which may be found. Discontinuous sucrose gradients, in particular a gradient as described above prepared from a 50% (wt./vol.) optionally buffered sucrose solution and a 20% (wt./vol.) buffered sucrose solution, furthermore exhibit suitable osmotic conditions in order not to deactivate any optionally present viral load. Ultracentrifugation according to process step d) ensures, for example, that the viral load originating from the pancreatin specimen is substantially quantitatively transferred from the pancreatin test sample supernatant into the discontinuous gradient medium. Substantially quantitative here means that an viral load previously added to a test sample is so completely recovered that the difference of titer in the added viral load and in that recovered after ultracentrifugation has been carried out is less than or equal to one half step of the base-ten logarithm of virus titer (= 0.5 log steps). The first ultracentrifugation according to process step d) furthermore ensures that the viral load is transported into a first target fraction which is sufficiently far away from the pancreatin test sample to permit mechanical separation thereof from the pancreatin test sample without it being possible for any remixing of the phases disruptive to the separation to occur.

Process step d) is conventionally carried out by introducing a volume of the highest concentration gradient medium into an ultracentrifuge tube over which is layered a volume of the next lower concentration gradient media. This process is repeated as many times as desired to obtain a multi-phase gradient medium with the final (top) layer being a volume of liquid pancreatin test sample suitable for centrifugation from which the viral load is to be separated. In the case of a two-phase gradient medium, the volume of the next lower concentration gradient medium is then immediately overlayeredwith a volume of a liquid pancreatin test sample or a pancreatin test sample suspension (pancreatin test sample volume) which is suitable for centrifugation. In the case of a two-phase gradient medium, this yields a sequence of phases in the ultracentrifuge tube from the top down of a first layer comprising the pancreatin test sample volume (top), then the volume of the lowest concentration gradient medium (middle; for example a 20% (wt./vol.) buffered sucrose solution) and finally the volume of the highest concentration gradient medium (bottom, covering the base of the ultracentrifuge tube; for example a 50% (wt./vol.) buffered sucrose solution). When overlaying the individual volumes, care must be taken to ensure that no turbulence or intermixing occurs at the respective boundaries.

The first target fraction in process step d) typically comprises (i) a part of the lowest concentration gradient medium sufficiently far away and remote from the pancreatin test sample volume and (ii) the complete volume of the next higher concentration gradient medium. In the case of a two-phase gradient medium the target fraction comprises, for example, a part of the lowest concentration gradient medium sufficiently far away and remote from the pancreatin test sample volume and the complete volume of the highest concentration gradient medium extending downward to the bottom of the ultracentrifuge tube optionally together with the pellet at the bottom of said tube.

When calculating the location of the first or second target fraction which is sufficiently remote from the pancreatin test sample volume thereby allowing for subsequent separation, it should be borne in mind that the calculated values for the position of the particles (i.e. the position of the viral particles separated from the pancreatin sample) usually denote the vertices of a Gaussian distribution. As such, the particles will be distributed both above and below their calculated position. It is thus necessary when determining the desired distance of the target fraction from the pancreatin test sample volume to include an additional margin to account for the Gaussian distribution of the particle locations. The viral load is conventionally transported into a target fraction which is sufficiently distant from the pancreatin test sample volume for subsequent separation if particles with a sedimentation constant of ≥ 120 S, in particular of ≥ 120 S to 5,000 S, have migrated from the pancreatin test sample volume at least 10 mm, for example at least 15 mm, at least 20 mm, at least 25 mm or at least 30 mm, into the lowest concentration gradient medium due to the ultracentrifugation. In the case of a two-phase gradient medium previously described, the lowest concentration gradient medium is the 20% (wt./vol.) buffered sucrose solution. In one variant of the ultracentrifugation step, substantially all particles with a sedimentation constant of ≥ 120 S, in particular of ≥ 120 S to 5,000 S, have completely passed through the lowest concentration gradient medium and are concentrated at the boundary layer to the next higher concentration gradient medium (i.e. on a "sucrose cushion"). The person skilled in the art is aware of suitable ways of calculating and implementing the corresponding conditions for ultracentrifugation. Suitable ultracentrifugation conditions may be determined based upon the characteristics of the virus(es) to be separated from the pancreatin sample (e.g., density and sedimentation constant), where applicable assuming per se known simplifications (see for example Lebowitz et al., "Modern analytical ultracentrifugation in protein science: A tutorial review"; Protein Science 11 (2002) 2067-2079).

Provided that the first ultracentrifugation is carried out using conventional volume ratios and in the discontinuous gradient medium, preferably in the two-phase discontinuous sucrose gradient, the viral load is conventionally transported into a first target fraction suitable for subsequent separation by the second ultracentrifugation, if the first ultracentrifugation is carried out for a duration of at least 1 hour, usually of at least 4 hours, like at least 9 hours, for example for a duration of 9-20 hours, in particular for a duration of 12-18 hours. A suitable relative centrifugal force for the first ultracentrifugation described herein is at least 50,000 × g and/or below 150,000 × g. In one embodiment of the first ultracentrifugation step, said step is carried out for a duration of 12-18 hours with a relative centrifugal force of 50,000-150,000 × g in volume ratios conventional for carrying out ultracentrifugation and in a gradient prepared from a 50% (wt./vol.) buffered sucrose solution and a 20% (wt./vol.) buffered sucrose solution. In another embodiment of the first ultracentrifugation step, said step is carried out for a duration of 15-17 hours with a relative centrifugal force of 70,000-120,000 × g in volume ratios conventional for carrying out ultracentrifugation and in a gradient prepared from a 50% (wt./vol.) buffered sucrose solution and a 20% (wt./vol.) buffered sucrose solution. Volume ratios conventional for carrying out ultracentrifugation are obtained, for example, if conventional ultracentrifuge tubes are used. Conventional ultracentrifuge tubes are here, for example, taken to be those with a volume of 10-50 ml, in particular 25-50 ml. Provided that a conventional ultracentrifuge tube is used in process step d), the volume of the highest concentration gradient medium (for example a 50% (wt./vol.) buffered sucrose solution) may amount, for example, to 2 ml, the volume of the next lower concentration gradient medium (for example a 20% (wt./vol.) buffered sucrose solution) may amount, for example, to 14 ml and the pancreatin test sample volume may amount, for example, to 17 ml.

The second ultracentrifugation is carried out in step e) using the first target fraction obtained in step d) and placing said first target fraction on a gradient medium cushion. Said gradient medium cushion is composed of e.g. the gradient medium of the same type as used in the first ultracentrifugation whereby said gradient medium is a higher concentration gradient medium than the concentration of the lowest concentration gradient component of the first target fraction. For example, in case the first ultracentrifugation is a discontinuous gradient centrifugation using the above described system of a 20 % wt./vol. buffered sucrose solution and a 50 % wt./vol. buffered sucrose solution, the resulting first target fraction is a system containing a buffered sucrose solution of a concentration of higher than 20 % wt./vol. and below 50 % wt./vol. In this case, the sucrose cushion may be a 50 % wt./vol. buffered sucrose solution. The first target fraction used in step d) usually also comprises any pellet optionally present after the first centrifugation.

The second ultracentrifugation is carried out for a duration of at least 1 hour, usually of at least 2 hours, for example for a duration of 2-8 hours, in particular for a duration of 3-6 hours. A suitable relative centrifugal force for the second ultracentrifugation described herein is at least 100,000 × g, for example above 150,000 × g, like 150,000-350,000 × g. In one embodiment of the second ultracentrifugation step, said step is carried out for a duration of 3-6 hours with a relative centrifugal force of 200,000-350,000 × g in volume ratios conventional for carrying out ultracentrifugation. In another embodiment of the second ultracentrifugation step, said step is carried out for a duration of 4.5-5.5 hours with a relative centrifugal force of 250,000-300,000 × g in volume ratios conventional for carrying out ultracentrifugation. Volume ratios conventional for carrying out ultracentrifugation are obtained, for example, if conventional ultracentrifuge tubes are used. Conventional ultracentrifuge tubes are here, for example, taken to be those with a volume of 10-15 ml, in particular 12-13 ml; an internal radius of 6-8 mm, in particular of 7 mm; and a height of 80-100 mm, in particular of 85-95 mm. Provided that a conventional ultracentrifuge tube is used in process step d), the volume of the gradient cushion (for example a 50% (wt./vol.) buffered sucrose solution) may amount, for example, to 0.5 ml, and the first target fraction volume may amount, for example, to 10 ml.

The present invention allows the detection of up to a detection limit of one infectious unit per gram pancreatin specimen used as starting material.

In a preferred variant of the embodiments of process steps d) and e), irrespective of the otherwise selected conditions, ultracentrifugation is carried out with cooling to a temperature of 0-15°C, preferably to a temperature of 4-10°C.

Conventional refrigerated centrifuges which may be used in this process step are known to the person skilled in the art. A conventional commercial ultracentrifuge is conventionally used in process steps d) and e), such as a coolable ultracentrifuge with a swinging bucket rotor, for example an ultracentrifuge from Sorvall^{®} with a model "TH-641" swinging-bucket rotor.

The above-stated description of the low-speed centrifugation steps and ultracentrifugation steps according to the invention may be scaled up or down to any desired extent by the person skilled in the art, in particular with the assistance of the further technical information stated in the description of the present invention.

In process steps d) and f), the first or second target fraction containing the viral load is quantitatively separated in each case from the pancreatin test sample supernatant and the first target fraction. Separation usually proceeds by placing a mark on the ultracentrifuge tube at the height of the previously determined boundary of the target fraction. The entire volume above this boundary is then separated from the remaining volume, for example by being aspirated. Aspiration may, for example, proceed with a conventional peristaltic pump, the tubing and capillaries of which have conveniently previously been sterilized. A suitable pumping rate is, for instance, a rate of 2 ml/minute. During aspiration, care should be taken to ensure that the peristaltic pump capillary is always located at the upper border of the liquid. The target fraction remaining in the ultracentrifuge tube may then be removed from the ultracentrifuge tube in per se known manner with a conventional single channel pipette, preferably with a sterile tip. Any sediment possibly still remaining in the ultracentrifuge tube may be removed at the same time, for example by being repeatedly drawn up and resuspended with the single channel pipette.

In one embodiment, the invention also provides an isolated second target fraction which may be produced according to process steps a)-f).

If a process for quantitatively determining the viral load of the pancreatin specimen is to be carried out, a process step g) follows process step f). In process step g), the viral load of the pancreatin specimen is quantitatively determined by determining the virus infection titer in the target fraction containing the viral load. Quantitative determination of the virus infection titer in the target fraction may here proceed in accordance with working processes known per se in virology, for example in accordance with the per se known principle of virus infection titer determination (= VITD).

The second target fraction may, for example, be diluted in a suitable ratio with a suitable cell culture medium or a saline solution, e.g. PBS, in order to obtain a virus determination test sample. Suitable cell culture media are those stated above to be usable, in each case as a function of the investigated virus species. In an embodiment of the invention to rule out false positive hits for virus infection, the diluted or undiluted target fraction may be filtered through a microfilter before the quantitative determination of the viral load is carried out in process step g).

A suitable dilution may, for example, be achieved by diluting the target fraction with the cell culture medium or the saline solution to the original volume of the pancreatin test sample supernatant used in process step d). Then, in a first step, a dilution series of the virus determination test samples may first be produced in per se known manner, for example in dilution steps of 1:2, 1:5 or 1:10 or also in combinations of these dilution steps, in order to carry out a quantitative VITD. Then, in a second step, a suitable cell suspension may be inoculated in per se known manner with the virus determination test samples of different concentrations from the dilution series, whereupon a cell layer is allowed to form on the virus determination test samples of different concentrations. To rule out false positive hits for virus infection which may be caused by the presence of microbials such as inert bacteria or mycoplasms, it is then usually expedient to filter the virus determination test samples before inoculating them onto detector cells. To this end, a virus determination test sample or a diluted virus determination test sample may be filtered through a filter of appropriate pore size, such as a microfilter, e.g. a microfilter of a pore size (i.e. the pore diameter) of from 0.1 to 10 µm (range limits included; = microfiltration), preferably of from 0.1 to 0.45 µm, usually a microfilter of a pore size (i.e. the pore diameter) of 0.1 µm. The filtrate may then be used as a test sample for further investigations. Then, in a third step, the inoculated test samples are read for their degree of infection depending on the manner in which their infection is indicated. Where, for example, CPE may be used as an indicator of infection of a cell layer, CPE is read in per se known manner after approximately 4-7 days. Titration (end-point dilution) of the virus determination test samples here permits a quantitative determination of the originally present infection dose. Titration conventionally proceeds by dilution by a factor of 10, i.e. based on the base-ten logarithm. In practice, the 50% infection dose (= ID₅₀) is usually calculated. In the case of parallel multiple batches, the identified ID₅₀ value then corresponds to that of the highest (reciprocal) dilution of the virus determination test sample at which a CPE is detectable in exactly half the batches. The results may optionally additionally be computationally corrected or interpolated in per se known manner. The most commonly used methods for virus titer calculation are those according to Spearman and Kärber (see C. Spearman, Br. J. Psychol. 2 (1908) 227-242 and G. Kärber, Arch. Exp. Path. Pharmak. 162 (1931) 480-483; also Bundesanzeiger [Federal gazette] no. 84, May 4 1994) or according to Reed and Muench (see Reed, L.J., Muench, H. Am. J. Hyg. 27 (1938) 493-497).

Other indicators of an infection of the cell layer may also be used, for example virus antigen induction or plaque induction. The person skilled in the art is familiar with these methods and their applications in the present case, for example from textbooks of virology such as "Medizinische Virologie" by H.W. Doerr and W.H. Gerlich, Georg Thieme Verlag Stuttgart, New York, 1st edition 2002 or in each case the most recent edition thereof.

The method according to the present invention is particularly useful for analyzing larger amounts of pancreatin specimens as starting material. Using large amounts of starting material usually allows to determine the viral load with a higher sensitivity, namely, to determine lower infectious units per gram. Thus, the method according to the present invention is particularly useful for starting materials of at least 5 g, e.g. of 8 g, 10 g or 12 g.

### EXAMPLES

All tasks stated in the following Examples were carried out under sterile conditions on a sterile workbench. The procedures conventional in virological laboratories, for example safety procedures must be observed. The following materials were inter alia used:
1. Antibiotic solution, 1.0 g of streptomycin sulfate and 1.2 g of penicillin are dissolved in 20 ml of twice-distilled water and filtered through a 0.2 µm filter. The filtrates are then divided into 1 ml aliquots and optionally stored at -20°C until use;
2. Dulbecco medium, cell culture medium for SK 6 cells, SPEV cells and MA 104 cells;
3. Single channel pipette, with sterile tips;
4. FCS, fetal calf serum (e.g. from Bio Whittaker; = serum).
5. Tissue culture flasks, sterile, area of flask base in each case 25, 75 or 175 cm²;
6. MEM, cell culture medium for PK-15 cells with 1.5 g/l sodium bicarbonate and 1 mM pyruvate;
7. Microtiter plates, sterile with 96 wells and lid;
8. PAN suspension ,10% pancreatin suspension; 8.0 g of porcine pancreatin (unless otherwise stated) weighed out under sterile conditions into a beaker, combined with 8 ml of antibiotic solution and (unless otherwise stated) 64.0 ml of the particular corresponding cell culture medium or PBS and (unless otherwise stated) suspended within 60 minutes in an ice bath with stirring;
9. Pardee buffer, Pardee's carbon dioxide buffer;
10. PBS, sterile "phosphate buffered saline" solution (pH 7.2);
11. Pipettes, sterile;
12. Pipette tips, sterile in sterile trays;
13. Plastics pouches, CO₂-impermeable with closure ("Anaerocult^{®}", from Merck);
14. Polyclonal anti-PPV antibody, fluorescein isothiocyanate (= FITC) conjugate, from NatuTec GmbH;
15. Tubes, sterile 15 and 50 ml;
16. Sucrose solution, 20%, PBS-buffered, sterile; concentration is adjusted in per se known manner with the assistance of a conventional refractometer;
17. Sucrose solutions, 50%, PBS-buffered, sterile; concentration is adjusted in per se known manner with the assistance of a conventional refractometer;
18. Screw-top tubes, sterile;
19. Trypsin solution, "TrypL Express^{®}", from INVITROGEN;
20. Peristaltic pump, from "ismaTec", pumping rate up to 5.8 ml/minute;
21. Refrigerated ultracentrifuge, "Sorvall^{®} Pro 80" with "TH-641" rotor;
22. Ultracentrifuge tubes, sterile, capacity 11 ml, dimensions 9 × 90 mm
23. Dilution blocks, 96 wells each of 1.0 ml;
24. MA-104 cells: supplied by FLI;
25. PK-15 cells: supplied by DARD;
26. SK-6 cells: supplied by FLI;
27. SPEV cells: supplied by FLI;
28. Cell suspensions of the SK 6, SPEV and PK-15 cells to be tested with 200,000 cells/ml in cell culture medium with 10% FCS;
29. Sterile Falcon microtubes, capacity 15 ml

### Example 1: Investigation of the harmful effect of pancreatin on various cell lines

For the purposes of detecting viruses in material test samples using cell cultures, the harmful effect of the pancreatin specimen to be investigated on the cells should be ascertained in order to be able to rule out false negative results when evaluating CPEs. As stated below, investigations to ascertain the harmful effect of a pancreatin test sample suspension were accordingly carried out on various cell lines.

0.5 ml portions of a PAN suspension produced as above were taken for testing the harmful effect and designated "pancreatin suspension test sample".

Low-speed centrifugation: The remaining PAN suspension was centrifuged for 15 minutes at 10,000 rpm (10,800 × g) and 4°C in a conventional refrigerated centrifuge (Biofuge^{®} 22R Heraeus SEPATECH^{®} with fixed-angle rotor no. 3745). The supernatant after low-speed centrifugation was then centrifuged for a further 15 minutes at 10,000 rpm and 4°C, and designated "supernatant after low-speed centrifugation", used for virus titration and ultracentrifugation. The two sediments obtained in each case after the low-speed centrifugations were combined (together 1 ml), resuspended in 9 ml of the respectively suitable cell culture medium and designated "sediment" or pellet.

The first ultracentrifugation: The test sample "supernatant after low-speed centrifugation" was subjected to a first ultracentrifugation in an ultracentrifuge. To this end, 2 ml of 50% (wt./vol.) sucrose solution was introduced by means of a pipette into the number of ultracentrifuge tubes necessary for carrying out testing. With the ultracentrifuge tube held at an oblique angle, 14 ml of a 20% sucrose solution was carefully placed on top of the 50% sucrose layer with, a dividing layer being discernible between the two solutions. A 17 ml layer of the "supernatant after low-speed centrifugation" test sample taken as stated above was then placed, again with care and avoiding turbulence and intermixing, onto the 20% (wt./vol.) sucrose solution. The ultracentrifuge tubes were then suspended in the ultracentrifuge rotor. To this end, the two ultracentrifuge tubes on the opposite sides of the rotor were in each case counterbalanced with PBS, inserted in the corresponding holders and tightly sealed with the associated lid. Once the holders had been inserted in the rotor, the test samples were centrifuged for 16 hours at 10°C and 22,000 rpm (87,000 × g). After ultracentrifugation, the ultracentrifuge tubes were removed from the holders on the sterile workbench and provided with a mark at a height of 5 cm, measured from the bottom of the ultracentrifuge tube. Using a peristaltic pump, the tubing and capillaries of which had previously been sterilized, the liquid above the mark was aspirated from the ultracentrifuge tube at a pumping rate of 2 ml/minute, the capillary always being located at the upper border of the liquid. This first fraction obtained in this manner was designated "upper fraction after first ultracentrifugation". The "lower fraction after first ultracentrifugation" (1.5 ml) remaining in the ultracentrifuge tube was in each case removed from the ultracentrifuge tube with the assistance of a single channel pipette. Any sediment possibly remaining on the bottom of the ultracentrifuge tube was resuspended by being repeatedly drawn up with the single channel pipette and likewise removed. The "lower fraction after first ultracentrifugation" was combined for all tubes and was used directly in the second ultracentrifugation. Prior to further processing, the resultant fractions were stored at 4°C or, in the case of extended storage, at -20°C.

The second centrifugation was carried out in two 11 ml-tubes for 5 hours at 4°C and at 272,000 x g. A gradient medium cushion of 50% (wt./vol.) sucrose solution was introduced into the tubes first (0.5 ml). Then, 5 ml of the first target fraction where layered onto the gradient cushion and ultracentrifugation was performed as indicated. After discarding the upper fraction, a volume of 1.5 ml was removed from the bottom of each tube excluding the pellet.

The test samples "pancreatin suspension test sample", "supernatant after low-speed centrifugation", "sediment" (after low-speed centrifugation), "upper fraction after first ultracentrifugation" and "lower fraction after first ultracentrifugation" (after being made up to 5.0 ml), as well as the "upper fraction after second ultracentrifugation" and "lower fraction after second ultracentrifugation" were then tested for their harmful effect with regard to various cell lines. To this end, dilution series of the test samples to be tested were in each case produced. All the test samples to be tested were further diluted by a factor of 2 from a dilution of 1:5 with the respectively suitable cell culture medium. In microtiter plates, there were added to 100 µl portions of cell suspension comprising PK-15, SPEV or SK 6 cells per well in 8 parallels, 100 µl of the test sample dilutions produced to in each case 100 µl of freshly produced cell suspension. When MA 104 cells were tested, microtiter plates with a 24 hour old cell layer were used. To this end, the cell culture medium was in each case removed from the wells and replaced with 100 µl of fresh cell culture medium without serum, so giving rise to final test sample dilutions of 1:10, 1:20, 1:40, 1:80, 1:160 etc. As a control, 100 µl of cell culture medium were introduced into eight wells of each microtiter plate instead of 100 µl of the dilution series. Pairs of plates together with a tube containing 4 ml of Pardee buffer and filter paper, were placed in air-tight pouches and tightly sealed with a sealing clip. The plates were then incubated at 36 ± 1°C for up to 7 days. Over the period of incubation, the plates were inspected daily by microscope for the extent of CPE, i.e. for cell lysis and/or degeneration of the cells and the absence of formation of a cell layer as a result of the harmful effect of the pancreatin. The final evaluation was carried out after seven days. The titration was repeated if cell degeneration had already occurred in the controls on the final reading.

The results of testing the different test samples for their harmful effect with regard to various cell lines revealed no harmful effect of the "lower fraction after second ultracentrifugation" derived from pancreatin on various cell lines.
The results demonstrated that, in the "lower fraction after first ultracentrifugation" and "the lower fraction after the second ultracentrifugation", which has been subjected to the first or first and second ultracentrifugation step according to the invention, respectively, and in which the viral load has been concentrated, there is a considerable reduction in the harmful effect towards the investigated cell lines in comparison with all the other test samples investigated.

### Example 2: Testing of different pancreatin charges on viral load

The two step ultracentrifugation procedure according to the present invention as described above was applied to isolate viruses from suspensions of different charges of pancreatin powder. The separation of the viruses from the pancreatin was the prerequisite for the detection of low virus loads by titration on susceptible cell lines:
- 8 g of drug substance were mixed with 8 ml of antibiotic solution (1.0 g streptomycin sulfate and 1.2 g Penicillin G per 20 ml of sterile PBS) and 64 ml of PBS and stirred in an ice water bath for 60 minutes
- the resulting suspension was centrifuged at 4°C for 15 minutes at 10,800 × g
- the supernatant after first centrifugation was centrifuged again at 4°C for 15 minutes at 10,800 × g

The resulting supernatant after low speed centrifugation was used for a first ultra centrifugation. The first ultra centrifugation was performed with a discontinuous sucrose gradient (four centrifugation tubes with 2 ml of 50% (wt./vol.) sucrose and 14 ml of 20% (wt./vol.) sucrose were covered with 17 ml of supernatant after low speed centrifugation). This technique allows the separation of virus particles from the main part of the pancreatin suspension at corresponding conditions. The viruses from virus/pancreatin suspension mixtures become concentrated in a border layer between 50% (wt./vol.) and 20% (wt./vol.) sucrose at appropriate centrifugation conditions. The soluble pancreatin components remain after centrifugation in the supernatant above the virus layer. The virus fraction is separated from the pancreatin layers by fractionated sucking off of the sucrose gradient. The resulting virus fraction was ultra centrifuged again for further concentration of viruses on a 50% (wt./vol.) sucrose cushion.

The first centrifugation was carried out in four 36 ml-tubes for 16 hours at 4°C and at 87,000 × g. After centrifugation a volume of 5 ml was removed from the bottom of each tube.

The second centrifugation was subsequently carried out in two 11 ml-tubes for 5 hours at 4 °C and at 272,000 × g. A volume of 1.5 ml was removed from the bottom of each tube. This resulted in a 3 ml virus concentrate which was obtained from 8 g of pancreatin powder. The samples were stored at -20°C.

### Infectivity assays

The infectivity assay is based on the assumption that a low virus load of a sample can be made visible by three consecutive passages of the sample through susceptible cell lines. The contained viruses can infect new cells and become amplified during these passages leading to an increased virus titre. In combination with the large volume plating technique it is possible to detect even a single infectious virus with a low amplification rate of 10 (10 viruses per 1 infectious virus within 6 days incubation) during three cell passages by the formation of about 1000 plaques on a cell lawn after the third passage.

The virus fractions extracted from the pancreatin samples were therefore applied to three consecutive passages on SK 6 cells for detection of infectious porcine Parvoviruses.

### 1. Passage (1ml virus fraction = 2,66 g pancreatin)

The third part of each individual virus concentrate (1 ml of 3 ml) was inoculated in a 75 cm² tissue culture flask containing 30 ml cell culture medium with 5% of FCS and a 24 h old subconfluent cell lawn of SK 6 cells. The cell culture flasks were incubated at 37 ± 1°C for 6 days.

The formation of cytopathic effects (plaques) was checked during the incubation and at the end of incubation time with the aid of an inverse microscope. The passage was finished with the creation of a virus/cell lysate by two freeze thaw cycles of the total tissue culture flask content. The virus cell lysate was applied to a low speed centrifugation (2,800 × g for 15 minutes) to create a cell free supernatant. This supernatant after low speed centrifugation (30 ml per lot) was filtrated through a filter with 0.1 µm pore size as a safety step to reduce a possible contamination of the lysate with mycoplasmas.

### 2. Passage

A 75 cm² tissue culture flask containing 22.5 ml cell culture medium with 5% FCS and a 24 h old cell lawn of SK 6 cells was prepared for the second passage of each lot. 7.5 ml of cell culture supernatant from first passage (= 25%) were added. Flasks were incubated for 6 days at 37 ± 1°C and were checked for cytopathic effects with the aid of an inverse microscope.

The virus/cell culture lysate (30 ml per lot) was achieved as described for the first passage.

### 3. Passage

A 75 cm² tissue culture flasks was prepared for each lot as described above. 7.5 ml of the virus/cell culture lysate from the 2. passage (= 25%) and 22.5 ml cell culture medium with 5% FCS were added per flask. Flasks were incubated for 6 days at 37 ± 1°C and inspected for cytopathic effects with the aid of an inverse microscope.

The virus/cell culture lysate (30 ml per lot) was achieved as described for the 1. and 2. passages.

### 4. Detection of porcine Parvovirus (PPV) by immuno-staining with FITC-conjugated anti-PPV antibodies

Micro titre plates with 96 wells and a 24 hour old cell lawn of SK 6 cells were prepared. 30 cavities were infected with 100 µl each of the virus/cell culture lysate from the 3. passage (= 10%) and incubated at 37 ± 1°C for 24 hours.

After incubation the cell culture supernatant was removed from the cells, and the cell lawn was fixed by an ice cold mixture of 80% acetone and 20% methanol. 100 µl of FITC-conjugated anti-PPV antibody solution per cavity were added and the plates were incubated for 45 minutes at 37 ± 1°C. After removing the antibody solution the cell lawn was washed 3 times with PBS and covered with 15 % glycerol in PBS. The detection of cells infected by Parvovirus was carried out with the aid of an inverse microscope in UV-light.

In parallel with the pancreatin samples porcine Parvovirus NADL-2 with known titre was titrated on the same microtitre plates as positive control.

All test items were passaged on SK 6 cells in parallel with the positive controls as described. By this way it was possible to compare the formation of plaques/cytopathic effects typical for porcine *Parvovirus* between pancreatin samples and positive controls.

The results of cultivation are shown in table 1.

**Table 1: Results of passaging the test items on SK 6 cells in cell tissue culture flasks**

| Lot number of pancreatin powder | Passaging of virus fractions of pancreatin samples on SK 6 cells in tissue culture flasks with inspection for cytopathic effects | | |
|---|---|---|---|
| | 1. passage | 2. passage | 3. passage |
| 0436 | no CPE detected | no CPE detected | no typical CPE detected but |
| | | | slight cell degeneration 6 days post infection |
| 0437 | no CPE detected | single plaques in cell lawn 4 days post infection, but non confluent CPE 6 days post infection | 30% of cell lawn with plaques 6 days post infection |
| 0438 | no CPE detected | 4 days post infection 80% of cell lawn with plaques, confluent CPE 6 days post infection | 2 days post infection visible plaque forma-tion; confluent CPE 4 days post infection |

At the first passage of the three test items no CPE was detected 6 days post infection.

No CPE was detectable during three passages of sample 0436. Only in the third passage of sample 0436 a slight cell degeneration which was however not identical with the observed cytopathic effects of samples 0437 and 0438 was found.

Single plaques in the cell lawn were observed during the second passage of sample 0437 four days post infection. 6 days post infection approximately 20 % of the cell lawn in the flask showed CPE. After third passage of the sample about 30% of the cell lawn showed cytopathic effects/plaques.

Single plaques were observed 3 days post infection during the second passage of sample 0438. In the course of further passages the plaque formation continued and led to nearly confluent CPE.

In the third passage a clear CPE was detected 2 days post infection. The incubation was finished 4 days post infection with a confluent CPE.

Subsequent to the third passage in tissue culture flasks the virus/cell culture lysate from third passage was transferred to micro titre plates with SK 6 cell lawn:
30 times 100 µl per lot into 30 wells in two parallels. A positive control of *porcine Parvovirus* NADL-2 was titrated on the same plates.

Plates were incubated at 37 ± 1°C, first plate for observing cytopathic effects and the second plate for detection of porcine *Parvovirus* by immunostaining.

The first plate was incubated for 6 days. At the end of incubation the samples 0437 and 0438 showed the same cytopathic effect as the positive control PPV

NADL-2. The sample 0436 did not show any CPE.

The culture supernatant from the second micro titre plate was removed 24 hours after starting the incubation: The short incubation time should reduce the ability of detection of cells with secondary infections by parvoviruses which were released from primary infected cells. Cell lawn was fixed and stained with an FITC-conjugated anti-PPV-antibody and checked for infected cells with the aid of an inverse microscope in UV-light. This corresponds with a plaque titration and gives the possibility to detect the virus titre of the third passage lysate.

The following results were obtained:
None of the 30 cavities which were infected with the cell free virus lysate from the third passage of sample 0436 contained distinct fluorescent cells.

Additional to the tested 3 ml (= 10%) further 30 x 100 µl of cell free virus lysate were transferred into micro titre plate with SK 6 cell lawn, incubated for 24 hours at 37 ± 1 °C, fixed and stained with anti-PPV antibody. Non- infected cells were found in these 30 additional cavities.

This result correlates with the results of passaging the sample 0436 in tissue culture flasks and after third passage in micro titre plates for detection of cytopathic effects.

In the case of samples 0437 and 0438 all 30 cavities which were stained with anti-PCV antibody contained distinct fluorescent cells such as the positive control PPV NADL-2.

As demonstrated above, the process according to the present invention allows one infectious unit to be identified per 1 g pancreatin, and, thus, provides a highly sensitive process for separating and determining viral load from a pancreatin specimen. Further, in contrast to processes based on molecular biology methods like detection of nucleic acid molecules based on amplification methods like PCR, the process according to the present invention allows determining infectious units. It is therefore possible to differentiate between infectious and non-infectious viral-load or total viral load determined by molecular biology techniques.

In a further embodiment, the passages and the detection can be performed stepwise (as described above) or in a parallel approach. Parallel approach means, that while one passage is performed the virus load of the previous passage can already be determined. Using this approach a result can be obtained from all passages and if the result is obtained from the first passage a result is achieved to an earlier point of time as using the stepwise approach. This might be of importance e.g. to increase time efficiency and/or e.g. for the quality assurance in manufacturing processes as the respective sample or product can be released at an earlier point of time.

## Claims

1. A process for separating a viral load from a pancreatin specimen, comprising the process steps:
a) producing a liquid pancreatin test sample suitable for centrifugation from the pancreatin specimen without in so doing changing the viral load thereof,
b) subjecting at least one defined part of the pancreatin test sample from process step a) to at least one low-speed centrifugation under conditions, under which viruses with sedimentation constants of ≥120 S do not yet form a pellet,
c) discarding any solid deposit optionally arising in process step b) during low-speed centrifugation and retaining a pancreatin test sample supernatant,
d) subjecting at least one defined part of the pancreatin test sample supernatant obtained in process step c) to a first ultracentrifugation in a discontinuous gradient medium, wherein the duration of the first ultracentrifugation and the relative centrifugal force for the first ultracentrifugation are selected such that the viral load is quantitatively transported out of the pancreatin test sample supernatant into a first target fraction situated above or in the boundary layer between the overlying, lowest concentration gradient component and the underlying, next higher concentration gradient component and quantitatively separating the first target fraction optionally including any pellet present after centrifugation containing the viral load from the pancreatin test sample supernatant;
e) subjecting the first target fraction obtained in step d) to a second ultracentrifugation whereby said second ultracentrifugation is carried out with a relative centrifugal force higher than the relative centrifugal force of the first ultracentrifugation on a gradient medium of the same type as used in the first ultracentrifugation whereby said gradient medium is a higher concentration gradient medium than the concentration of the lowest concentration gradient component in the first target fraction, and
f) obtaining a second target fraction containing the viral load by discarding the upper 75% vol./vol. liquid or more of the upper layer corresponding to the first target fraction and obtaining the lower fraction corresponding to the lower 25% vol./vol. liquid or less of the upper layer and the complete layer of the higher concentration gradient medium excluding any pellet optionally present after centrifugation.

2. The process as claimed in claim 1, in which additionally in a process step g) after process step f) a quantitative determination of the viral load of the pancreatin specimen is carried out by determining the virus infection titer in the second target fraction containing the viral load.

3. The process as claimed in claim 2, in which the diluted or undiluted second target fraction is filtered through a microfilter before the quantitative determination of the viral load is carried out.

4. The process as claimed in claim 1, in which in process step a) the pancreatin test sample is produced as a pancreatin test sample suspension by combining the pancreatin specimen with a cell culture medium which is suitable for the cell line used to culture the virus type to be investigated or with a saline solution, and with one or more antibiotics.

5. The process as claimed in claim 4, in which at least one step of steps a) to f) are carried out with cooling to a temperature of 0-15°C.

6. The process as claimed in claim 1, in which low-speed centrifugations in process step b) are in each case carried out with a relative centrifugal force of less than 15,000 × g.

7. The process as claimed in claim 1, in which low-speed centrifugations in process step b) are in each case carried out with a relative centrifugal force of 8,000-15,000 × g.

8. The process as claimed in claim 1, in which low-speed centrifugations in process step b) are carried out for a duration of at least 5 minutes.

9. The process as claimed in claim 1, in which the first ultracentrifugation in process step d) is carried out for duration of at least 9 hours.

10. The process as claimed in claim 1, where in the first ultracentrifugation in process step d) the relative centrifugal force is below 150,000 × g.

11. The process as claimed in claim 1, in which the second ultracentrifugation in process step e) is carried out for a duration of at least 2 hours.

12. The process as claimed in claim 11, where in the second ultracentrifugation in process step e) the relative centrifugal force is above 150,000 × g.

13. The process as claimed in claim 1, in which the discontinuous gradient medium introduced in process step d) is a discontinuous two-phase sucrose gradient.

14. The process as claimed in claim 13, in which the discontinuous gradient medium is a gradient comprising a 50% wt./vol. buffered sucrose solution and a 20% wt./vol. buffered sucrose solution.

15. The process as claimed in claim 1, in which the higher concentration gradient medium in process step e) is a gradient medium of 50% wt./vol. buffered sucrose solution.

16. The process as claimed in claim 1, in which the pancreatin specimen is a porcine pancreatin specimen.

17. The process as claimed in claim 1, in which the viral load of the pancreatin test sample comprises bovine rotavirus A, encephalomyocarditis virus, porcine circovirus, porcine parvovirus, porcine rotavirus A, porcine teschovirus, porcine hepatitis E virus and/or swine vesicular disease virus.

18. The process according to any one of the preceding claims wherein the pancreatin specimen used as starting material comprises at least 5 g pancreatin.

## Patentansprüche

1. Verfahren zum Abtrennen einer Viruslast aus einer Pankreatinprobe, umfassend die Verfahrensschritte:
a) Herstellen einer zentrifugierbaren flüssigen Pankreatintestprobe aus der Pankreatinprobe, ohne dass dadurch die Viruslast davon verändert wird,
b) Unterziehen wenigstens eines definierten Teils der Pankreatintestprobe aus dem Verfahrensschritt a) wenigstens einer Zentrifugation bei niedriger Drehzahl unter Bedingungen, bei denen Viren mit Sedimentationskonstanten ≥ 120 S noch kein Pellet ausbilden,
c) Verwerfen jeglicher festen Ablagerung, die möglicherweise im Verfahrensschritt b) während der Zentrifugation bei niedriger Drehzahl entsteht, und Behalten eines Pankreatintestprobenüberstandes,
d) Unterziehen wenigstens eines definierten Teils des im Verfahrensschritt c) erhaltenen Pankreatintestprobenüberstandes einer ersten Ultrazentrifugation in einem diskontinuierlichen Gradientenmedium, wobei die Dauer der ersten Ultrazentrifugation und die relative Zentrifugalkraft für die erste Ultrazentrifugation so ausgewählt sind, dass die Viruslast quantitativ aus dem Pankreatintestprobenüberstand in eine erste Zielfraktion transportiert wird, die oberhalb oder in der Grenzschicht zwischen der überlagernden Schicht mit der niedrigsten Konzentrationsgradientenkomponente und der unterliegenden Schicht mit der nächsthöheren Konzentrationsgradientenkomponente angeordnet ist, und quantitatives Abtrennen der ersten Zielfraktion, die eventuell ein Pellet enthält, das nach der Zentrifugation vorhanden ist und die Viruslast enthält, von dem Pankreatintestprobenüberstand,
e) Unterziehen der in Schritt d) erhaltenen ersten Zielfraktion einer zweiten Ultrazentrifugation, wobei die zweite Ultrazentrifugation mit einer relativen Zentrifugalkraft, die höher ist als die relative Zentrifugalkraft der ersten Ultrazentrifugation, in einem Gradientenmedium desselben Typs, das in der ersten Ultrazentrifugation verwendet wurde, ausgeführt wird, wobei das Gradientenmedium ein höheres Konzentrationsgradientenmedium ist als die Konzentration der niedrigsten Konzentrationsgradientenkomponente in der ersten Zielfraktion, und
f) Erhalten einer zweiten Zielfraktion, die die Viruslast enthält, durch Verwerfen der oberen 75 % Vol./Vol. Flüssigkeit oder mehr der oberen Schicht, die der ersten Zielfraktion entspricht, und Erhalten der unteren Fraktion, die den unteren 25 % Vol./Vol. Flüssigkeit oder weniger der oberen Schicht entspricht, und wobei die komplette Schicht des höheren Konzentrationsgradientenmediums jegliches Pellet, das enventuell nach der Zentrifugation vorhanden ist, ausgrenzt.

2. Verfahren nach Anspruch 1, in dem zusätzlich in einem Verfahrensschritt g) nach dem Verfahrensschritt f) eine quantitative Bestimmung der Viruslast der Pankreatinprobe mittels Bestimmen des Virusinfektionstiters in der zweiten Zielfraktion, die die Viruslast enthält, ausgeführt wird.

3. Verfahren nach Anspruch 2, wobei die verdünnte oder unverdünnte zweite Zielfraktion durch einen Mikrofilter gefiltert wird, bevor die quantitative Bestimmung der Viruslast ausgeführt wird.

4. Verfahren nach Anspruch 1, wobei die Pankreatintestprobe im Verfahrensschritt a) als eine Pankreatintestprobensuspension mittels Kombinieren der Pankreatinprobe mit einem Zellkulturmedium, welches für die Zelllinie geeignet ist, die verwendet wird, um den zu untersuchenden Virustyp zu kultivieren, oder mit einer Salinenlösung und mit einem oder mehreren Antibiotikum/Antibiotika hergestellt wird.

5. Verfahren nach Anspruch 4, wobei wenigstens ein Schritt der Schritte a) bis f) unter Abkühlen auf eine Temperatur von 0-15° C ausgeführt wird.

6. Verfahren nach Anspruch 1, wobei die Zentrifugationen bei niedriger Drehzahl in Verfahrensschritt b) in jedem Fall mit einer relativen Zentrifugalkraft von weniger als 15.000 g ausgeführt werden.

7. Verfahren nach Anspruch 1, wobei die Zentrifugationen bei niedriger Drehzahl in Verfahrensschritt b) in jedem Fall mit einer relativen Zentrifugalkraft von 8.000 bis 15.000 g ausgeführt werden.

8. Verfahren nach Anspruch 1, wobei die Zentrifugationen bei langsamer Drehzahl in Verfahrensschritt b) für eine Dauer von wenigstens 5 Minuten ausgeführt werden.

9. Verfahren nach Anspruch 1, wobei die erste Ultrazentrifugation in Verfahrensschritt d) für eine Dauer von wenigstens 9 Stunden ausgeführt wird.

10. Verfahren nach Anspruch 1, wobei die relative Zentrifugalkraft in der ersten Ultrazentrifugation in Verfahrensschritt d) unter 150.000 g beträgt.

11. Verfahren nach Anspruch 1, wobei die zweite Ultrazentrifugation in Verfahrensschritt e) für eine Dauer von wenigstens 2 Stunden ausgeführt wird.

12. Verfahren nach Anspruch 11, wobei die relative Zentrifugalkraft in der zweiten Ultrazentrifugation in Verfahrensschritt e) über 150.000 g beträgt.

13. Verfahren nach Anspruch 1, wobei das in Verfahrensschritt d) eingeführte diskontinuierliche Gradientenmedium ein diskontinuierlicher Zwei-Phasen-Saccharosegradient ist.

14. Verfahren nach Anspruch 13, wobei das diskontinuierliche Gradientenmedium ein Gradient ist, der eine 50 % wt./vol. gepufferte Saccharoselösung und eine 20 % wt./vol. gepufferte Saccharoselösung umfasst.

15. Verfahren nach Anspruch 1, wobei das höhere Konzentrationsgradientenmedium in Verfahrensschritt e) ein Gradientenmedium von 50 % wt./vol. gepufferte Saccharoselösung ist.

16. Verfahren nach Anspruch 1, wobei die Pankreatinprobe eine Schweinepankreatinprobe ist.

17. Verfahren nach Anspruch 1, wobei die Viruslast der Pankreatintestprobe bovines Rotavirus A, Encephalomyocarditis-Virus, porcines Circovirus, porcines Parvovirus, porcines Rotavirus A, porcines Teschovirus, porcines Hepatitis-E-Virus und/oder das Virus der vesikulären Schweinekrankheit umfasst.

18. Verfahren nach einem der vorhergehenden Ansprüche, wobei die als Startmaterial verwendete Pankreatinprobe wenigstens 5 g Pankreatin umfasst.

## Revendications

1. Procédé de séparation d'une charge virale d'un échantillon de pancréatine, comprenant les étapes de procédé consistant :
a) à produire un échantillon test de pancréatine liquide approprié pour une centrifugation à partir de l'échantillon de pancréatine sans modifier la charge virale de celui-ci,
b) à soumettre au moins une partie définie de l'échantillon test de pancréatine de l'étape de procédé a) à au moins une centrifugation à faible vitesse dans des conditions dans lesquelles des virus avec des constantes de sédimentation ≥ 120 S ne forment pas encore une boulette,
c) à mettre aux déchets tout dépôt solide éventuellement produit dans l'étape de procédé b) pendant la centrifugation à faible vitesse et à retenir une matière surnageante d'échantillon test de pancréatine,
d) à soumettre au moins une partie définie de la matière surnageante d'échantillon test de pancréatine obtenue dans l'étape de procédé c) à une première ultracentrifugation dans un milieu de gradient discontinu, dans lequel la durée de la première ultracentrifugation et la force centrifuge relative pour la première ultracentrifugation sont choisies de sorte que la charge virale est quantitativement transportée à l'extérieur de la matière surnageante d'échantillon test de pancréatine dans une première fraction ciblée située au-dessus ou dans la couche limite entre le constituant de gradient de concentration la plus faible, sus-jacent et le constituant de gradient de concentration plus élevée, sous-jacent et à séparer quantitativement la première fraction ciblée incluant éventuellement toute boulette présente après la centrifugation contenant la charge virale de la matière surnageante d'échantillon test de pancréatine ;
e) à soumettre la première fraction ciblée obtenue dans l'étape d) à une seconde ultracentrifugation, sur quoi ladite seconde ultracentrifugation est réalisée avec une force centrifuge relative supérieure à la force centrifuge relative de la première ultracentrifugation sur un milieu de gradient du même type que celui utilisé dans la première ultracentrifugation, sur quoi ledit milieu de gradient est un milieu de gradient de concentration plus élevée que la concentration du constituant de gradient de concentration la plus faible dans la première fraction ciblée, et
f) à obtenir une seconde fraction cibée contenant la charge virale en mettant aux déchets le liquide supérieur à 75 % vol./vol. ou plus de la couche supérieure correspondant à la première fraction ciblée et à obtenir la fraction inférieure correspondant au liquide inférieur à 25 % vol./vol. ou moins de la couche supérieure et la couche complète du milieu de gradient de concentration plus élevée excluant toute boulette éventuellement présente après centrifugation.

2. Procédé selon la revendication 1, dans lequel de plus dans une étape de procédé g) après l'étape de procédé f) une détermination quantitative de la charge virale de l'échantillon de pancréatine est réalisée en déterminant le titre d'infection par le virus dans la seconde fraction ciblée contenant la charge virale.

3. Procédé selon la revendication 2, dans lequel la seconde fraction ciblée diluée ou non diluée est filtrée à travers un microfiltre avant que la détermination quantitative de la charge virale soit réalisée.

4. Procédé selon la revendication 1, dans lequel dans l'étape de procédé a) l'échantillon test de pancréatine est produit comme une suspension d'échantillon test de pancréatine en combinant l'échantillon de pancréatine avec un milieu de culture de cellules qui est approprié pour la ligne de cellules utilisée pour mettre le type de virus à examiner en culture ou avec une solution saline, et avec un ou plusieurs antibiotiques.

5. Procédé selon la revendication 4, dans lequel au moins une étape des étapes a) à f) est réalisée avec un refroidissement à une température de 0-15°C.

6. Procédé selon la revendication 1, dans lequel des centrifugations à faible vitesse dans l'étape de procédé b) sont réalisées dans chaque cas avec une force centrifuge relative inférieure à 15 000 × g.

7. Procédé selon la revendication 1, dans lequel des centrifugations à faible vitesse dans l'étape de procédé b) sont réalisées dans chaque cas avec une force centrifuge relative de 8 000-15 000 x g.

8. Procédé selon la revendication 1, dans lequel des centrifugations à faible vitesse dans l'étape de procédé b) sont réalisées sur une durée d'au moins 5 minutes.

9. Procédé selon la revendication 1, dans lequel la première ultracentrifugation dans l'étape de procédé d) est réalisée sur une durée d'au moins 9 heures.

10. Procédé selon la revendication 1, dans lequel dans la première ultracentrifugation dans l'étape de procédé d) la force centrifuge relative est inférieure à 150 000 x g.

11. Procédé selon la revendication 1, dans lequel la seconde ultracentrifugation dans l'étape de procédé e) est réalisée sur une durée d'au moins 2 heures.

12. Procédé selon la revendication 11, dans lequel dans la seconde ultracentrifugation dans l'étape de procédé e) la force centrifuge relative est supérieure à 150 000 x g.

13. Procédé selon la revendication 1, dans lequel le milieu de gradient discontinu introduit dans l'étape de procédé d) est un gradient de saccharose à deux phases discontinu.

14. Procédé selon la revendication 13, dans lequel le milieu de gradient discontinu est un gradient comprenant une solution de saccharose tamponnée à 50 % en poids/volume et une solution de saccharose tamponnée à 20 % en poids/volume.

15. Procédé selon la revendication 1, dans lequel le milieu de gradient de concentration plus élevée dans l'étape de procédé e) est un milieu de gradient de solution de saccharose tamponnée à 50 % en poids/volume.

16. Procédé selon la revendication 1, dans lequel l'échantillon de pancréatine est un échantillon de pancréatine porcine.

17. Procédé selon la revendication 1, dans lequel la charge virale de l'échantillon test de pancréatine comprend le rotavirus bovin A, le virus d'encéphalomyocardie, le circovirus porcin, le parvovirus porcin, le rotavirus porcin A, le teschovirus porcin, le virus d'hépatite E porcine et/ou le virus de maladie vésiculaire chez le porc.

18. Procédé selon l'une quelconque des revendications précédentes, dans lequel l'échantillon de pancréatine utilisé comme matière de départ comprend au moins 5 g de pancréatine.
